Europäisches Patentamt

European Patent Office

Office européen des brevets

(19)

Veröffentlichungsnummer: **0 294 535**

**A1**

## (12) EUROPÄISCHE PATENTANMELDUNG

(21) Anmeldenummer: **87890127.1**

(22) Anmeldetag: **09.06.87**

(51) Int. Cl.4: **G01N 33/569 , C12N 11/00 , C12N 1/10**

(43) Veröffentlichungstag der Anmeldung:
**14.12.88 Patentblatt 88/50**

(84) Benannte Vertragsstaaten:
**AT BE CH DE ES FR GB GR IT LI LU NL SE**

(71) Anmelder: **Waldheim Pharmazeutika Gesellschaft m.b.H.**
**Landeggerstrasse 7**
**A-2491 Neufeld/Leitha (Burgenland)(AT)**

(72) Erfinder: **Pöckl, Erich E., Dr**
**Pöttschingerstrasse 23**
**A-7201 Neudörfl/L(AT)**
Erfinder: **Scheiner, Otto, Dr.**
**Hohe Wand Strasse 40**
**A-2344 Maria Enzersdorf(AT)**
Erfinder: **Frantsits, Werner J., Dipl.-Ing**
**Boltzmanngasse 11**
**A-1090 Wien(AT)**
Erfinder: **Wiedermann, Gerhard, Prof. Dr.**
**Trazerberggasse 4**
**A-1130 Wien(AT)**
Erfinder: **Stemberger, Heinrich, Dr.**
**Ziegelofengasse 15**
**A-3400 Klosterneuburg(AT)**

(74) Vertreter: **Beer, Manfred, Dipl.-Ing. et al**
**Patentanwälte Dipl.-Ing. Otto Beer, Dipl.-Ing.**
**Manfred Beer Lindengasse 8, Postfach 462**
**A-1071 Wien(AT)**

(54) **Verfahren zum Nachweis von Antikörpern, und Mittel zum Nachweis von Antikörpern und Verfahren zur Herstellung des Mittels.**

(57) Zum Nachweis von Antikörpern gegen Entamoeba histolytica wird die Probe mit Entamoeba histolytica-Antigen, das auf einem Träger fixiert ist, kontaktiert, diejenigen Antikörper, die mit dem fixierten Entamoeba histolytica-Antigen keine Antikörper-Antigen-Reaktion eingegangen sind, werden entfernt, der erhaltene, immobilisierte Komplex mit einem Antikörper, der mit einem Enzym konjugiert ist, kontaktiert, der nicht angelagerte, mit dem Enzym konjugierte Antikörper entfernt und die enzymatische Aktivität an Hand der Farbreaktion eines Substrates bestimmt.

Ein Mittel zur Durchführung des Verfahrens weist einen Träger auf, der mit einem Entamoeba histolytica-Antigen beschichtet ist, das durch Kultivieren von Trophozoiten von Entamoeba histolytica insbesondere des Stammes A3 gewonnen ist.

Ein besonders geeignetes Verfahren zur Herstellung des Testmittels wird angegeben.

EP 0 294 535 A1

# Verfahren zum Nachweis von Antikörpern, und Mittel zum Nachweis von Antikörpern und Verfahren zur Herstellung des Mittels

Die vorliegende Erfindung betrifft ein nach einer abgewandelten ELISA-Technik (ELISA = Enzyme Linked Immunosorbent Assay) arbeitendes Verfahren zur Bestimmung von Antikörpern gegen Entamoeba histolytica in Proben, insbesondere in Blutproben.

Entamoeba histolytica ist weltweit verbreitet, tritt in warmen Ländern endemisch - epidemisch auf (bis zu 50 % der Bevölkerung), in gemäßigten Klimaten sporadisch (bis zu 5 % der Bevölkerung).

Die serologische Bestimmung von Antikörpern gegen Entamoeba histolytica ist besonders für die Diagnose des Amöben- Leber-Abszesses und der invasiven intestinalen Form der Amöbiasis sowie der Amöbenperitonitis-Perikarditis und metastatischer Amöbenabsiedlungen in anderen Organen (Gehirn, Milz) von Bedeutung, insbesondere da diese meist mit unklarer klinischer Symptomatik einhergehen. Besonders wichtig ist daher eine spezifische Differentialdiagnose.

Bisher sind folgende Tests in der Amöbenserologie vorgeschlagen worden:

Der erste Complement-Fixationstest für Amöben wurde durch Izar G. 1914, Studien über Amoebenenteritis, Mitteilung IV. Über das Vorkommen spezifischer Antikörper im Serum von Amoebenruhrkranken, Arch. f. Schiffs- und Tropen-Hygiene, 18 (suppl.): 45-79 und Scalar L., 1921, Contributo allo studio della deviazione del complemento nella dissentieria amebica. Riforma Med., 37: 103-104 entwickelt; er beruht auf der Koppelung eines Antigen-Antikörper-Systems mit einem hämolytischen System als Indikator. Bei erfolgter Antigen-Antikörper-Reaktion wird Complement fi xiert, so daß keine Hämolyse auftritt.

Wagener E. H., 1924, A precipitin test in experimental amoebic dysentery in cats, Univ. Calif. Publ. Zool. 27: 15-20 verwendete den Precipitin-Test, wobei durch Diffusion von Antigen und Antikörper sichtbare Präzipitate entstehen. Eine Modernisierung des Precipitin-Tests wurde von Nakamura M. und Baker E.E. 1957, Agar diffusion precipitin technic for the detection of antibodies against Entamoeba histolytica, Bacteriol. Proc. (M 105) p. 95 sowie Atchley, F.O., Auerheiner, A.H. und Wasley, M.A., 1963, Precipitate patterns in agar gel with sera from human amebiasis and Entamoeba histolytica antigen, J. Parasitol., 49: 313-315 durch Verwendung von Agar Gel-Diffusionstechnik und von Siddiqui, W.A., 1961, Demonstration of antigen-antibody reactions with a monobacterial culture of Entamoeba histolytica in agar-gel and on cellulose acetate membrane, J. Parasitol 47, 371-372 zusätzlich durch Celluloseacetate-membrantechnik durchgeführt.

Die indirekte Haemagglutination ("IHA" Kessel, J.F., Lewis, W.P., Ma, S. und Kim H., 1961, Preliminary report on a haemagglutinationstest for Entamoebae, Proc. Soc. Exper. Biol. u. Med. 106: 409-413) verwendet sensibilisierte humane Erythrozyten der Blutgruppe O, Rh + und läßt diese mit Serum reagieren. Sind Antikörper vorhanden, kommt es zur Haemagglutination.

Iris, M. Krupp, Glutaraldehyde - treated cells in the indirect Haemagglutinationstest for Amebiasis, Am. J. Trop. Med. 0. Hyg. Vol. 18, No. 5 verbesserte diese Methode durch Glutaraldehyd- Fixation der Erythrozyten.

Der Latexagglutinationstest (William Anthony Sodeman, Jr., M.D. und Marsha C. Dowda, Rapid Serological Methods for the Demonstration of Entamoeba Histolytica Activity, Gastroenterology 65: 604-607, 1973; M.N. Morris, R. Elsdon-Dew, S. J. Powell, Latex Agglutination Test for Invasive Amoebiasis, The Lancet, June 27, 1970, 1262-1263) verwendet Antigen-beschichtete Latexpartikel. Positive Ergebnisse erscheinen als Agglutination.

Diese Tests verwendeten mehr oder weniger gereinigtes Antigen, modernere Tests wie ELISA, CIE, IFT verwenden gereinigte Antigene.

Immunfluoreszenztest (IFT):

Die Amöben wurden in gemischter Bakterienflora gezüchtet und durch Sedimentationsvorgänge partiell gereinigt. Anschließend wurden diese auf Objektträgern fixiert. Nach Aufbringen von Patientensera und fluoreszierendem Konjugat auf die Objektträger wird mittels Fluoreszenzmikroskop bewertet (Antikörper-hältige Sera verursachen Fluoreszenz (Pierre Ambroise-Thomas und Thai Kien Truong, Fluorescent Antibody Test in Amebiasis, Clinical Applications, Am. J. Trop. Med. Hyg. Vol. 21, No. 6, 907-912)).

EP 0 294 535 A1

Counterimmunelektrophorese (CIE):

Dieser Test verwendet das Prinzip der Immunpräzipitation und der Elektrophorese. Als Trägermedium fungiert ein Ionenagar auf dem Objektträger. Die Anodenseite wird mit Serum, die Kathodenseite mit Antigen gefüllt. Durch Anlegung eines elektrischen Feldes wandern die Antikörper zur Kathode, und die Antigene zur Anode. Wo sie sich treffen, kommt es zu Immunpräzipitation. Als Antigen wurden von R. Disko und Th. Schinkl, Zur Diagnose des Amöben-Leberabszesses, Munch med. Wschr. 121 (1979) Nr. 46, 1536-1538 axenisch kultivierte HK 9 Trophozoiten verwendet. Iris M. Krupp (Comparison of Counterimmunelectrophoresis with other serologic Tests in the Diagnosis of Amebiasis, Am. J. Trop. Med. Hyg. Vol. 23 No. 1, 27-30) bediente sich axenisch gezogener, mit Ultraschall behandelter Entamoeba histolytica (Stamm 200).


Enzyme-linked-immunosorbent assay (ELISA):

Es sind bereits mehrere ELISAs in Verwendung, die sich vor allem in ihren Antigenen unterscheiden, woraus sich eine unterschiedliche Spezifität und Sensitivität der Testsysteme ergibt.

F. Speiser (Anwendung einer Enzym-Immunmethode (ELISA) für die Serologie der Amöbiase, Schweiz. med. Wschr. 110, 4o4-4o7, 1980) beschreibt die Verwendung von ICN HK 9 Amöben AG, A. Voller, Ann Bartlett und D.E. Bridwell, Enzyme immunoassays for parasitic diseases, Trans. R. Soc. Trop. Med. Hyg., Vol. 70, No. 2, 1976, 98-105) die Verwendung axenisch kultivierter mit Ultraschall behandelter Entamoeba histolytica.

P. Felgner, Stepless Antibody Determination with the Stick-ELISA Technique Results Expressed as Multiple of Normal Activity (MONA), Zbl. Bakt. Hyg. I Akt A 242, 100-105 and Michael Funke, Paul Felgner und Rudolf Geister, Quantification of Amebae Specific Antibodies as "Multiple of Normal Activity (MONA)" with a Standardized Enzyme Immunoassay (EIA), Zbl. Bakt. Hyg. I Abt. Orig. A 251, 126-133 (1981) beschreiben einen Stick-ELISA mit Polystyrolstab als Antigen- Träger.

Die bisher in Verwendung stehenden Tests zeigten keine ausreichende Spezifität und Sensitivität für die sichere Diagnose der invasiven Amöbiasis. Die in der Amöbenserologie verwendeten Verfahren haben außer ELISA und IFT aufgrund ihrer komplizierten Handhabung oder unsicheren Aussagekraft nie größere Bedeutung erlangt. In zahlreichen epidemiologischen Studien (Iris M. Krupp, Comparison of Counterimmunelectrophoresis with other serologic Tests in the Diagnosis of Amebiasis, Am. J. Trop. Med. Hyg. Vol. 23 No. 1 27-30) erwiesen sich die ELISAs dem IFT überlegen, auch ELISAs zeigten jedoch nicht die für eine sichere Differentialdiagnose gewünschte Spezifität und Sensitivität. Ihnen allen ist die Verwendung eines hauptsächlich aus Zytoplasmaprotein bestehenden Antigens und der alleinige Nachweis von Antikörpern der IgG-Klasse gemeinsam.

Der Erfindung liegt die Aufgabe zugrunde, ein Verfahren der eingangs genannten Gattung zur Verfügung zu stellen, nach dem der sichere Nachweis von Amöbeninfektionen möglich ist. Gemäß der Erfindung wird diese Aufgabe dadurch gelöst, daß man zum Nachweis von Antikörpern gegen Entamoeba histolytica folgende Verfahrensschritte anwendet:

a) Kontaktieren der Probe mit Entamoeba histolytica-Antigen, das in einem später näher beschriebenen Verfahren erzeugt wird und auf einem Träger fixiert ist,

b) Entfernung derjenigen Antikörper, die mit dem fixierten Entamoeba histolytica-Antigen keine Verbindung eingegangen sind,

c) Kontaktieren des so behandelten Trägers mit einem Antikörper, insbesondere einem Anti-human-immunglobulin G, Anti-human-immunglobulin M, Anti-human-immunglobulin A, der mit alkalischer Phosphatase (z.B. Meerrettich-Peroxidase, Glucose-6- Phosphatase) konjugiert ist, und der sich ausschließlich an den Antigen-Antikörper-Komplex anlagert,

d) Entfernen des nicht angelagerten Konjugats,

e) Reaktion eines Enzyms mit dem geeigneten Substrat (z.B. o-Phenylendiamin 2HCl, p-Nitrophenylphosphat),

f) Stoppen der Reaktion,

g) Prüfen der so behandelten Probe hinsichtlich des Auftretens einer Verfärbung.

Das erfindungsgemäße Verfahren dieser ELISA-Technik er möglicht eine schnelle und vor allem sichere Diagnostik des Vorhandenseins von Antikörpern der Klasse IgG, IgM oder IgA gegen Entamoeba histolytica in Proben, im speziellen in Blutproben.

Bevorzugt ist dabei erfindungsgemäß, wenn man in Stufe c) mit Meerrettich-Peroxidase oder Glucose-6-Phosphatase konjugiertes Anti-Human Immunglobulin verwendet.

Die in Stufe c) des erfindungsgemäßen Verfahrens bevorzugt verwendeten Anti-Human Immunoglobuline sind beispielsweise eine Anti-Human IgG, IgM oder IgA.

Günstig ist es, wenn man als Substrat in Stufe e) o-Phenylendiamin.2HCL oder p-Nitrophenylphosphat verwendet.

Die Erfindung betrifft weiters ein Mittel zur Durchführung des Verfahrens der Erfindung. Dieses Mittel besteht aus einem Träger, der vorzugsweise eine Mikrotiterplatte ist und der mit einem Entamoeba histolytica-Antigen beschichtet ist.

Schließlich umfaßt die Erfindung auch ein Verfahren zur Herstellung des Mittels nach der Erfindung. Dieses Herstellungsverfahren zeichnet sich erfindungsgemäß dadurch aus, daß man Trophozoiten von Entamoeba histolytica, insbesondere des Stammes A3 kultiviert, daß man aus der Kulturbrühe Entamoeba histolytica-Antigen gewinnt und mit dem gewonnenen Antigen einen Träger beschichtet.

Mit Vorteil kann bei der Herstellung des Mittels erfindungsgemäß so vorgegangen werden, daß man die Trophozoiten von Entamoeba hystolytica monoxenisch, insbesondere mit Crethidium in einem Medium, vorzugsweise im Medium TYI-S$_3$ nach Diamond kultiviert.

Zur Gewinnung des Antigens bewährt es sich im Rahmen der Erfindung, daß man die Kulturbrühe homogenisiert und zen trifugiert und daß man den Träger mit dem derart gewonnenen Niederschlag (Membranfraktion), nach dem dieser in einer Pufferlösung, vorzugsweise einen 50 mM $CO_3^{--}/HCO_3^{-}$-Puffer, aufgenommen worden ist, beschichtet.

Das erfindungsgemäße Mittel ist ganz besonders zur Durchführung von Antikörper-Reihen-Untersuchungen geeignet. Im einzelnen kann das Mittel gemäß der Erfindung hergestellt werden, indem man

h) Trophozoiten von Entamoeba histolytica des Stammes A3 monoxe nisch (mit Crethidium) in Medium TYI-S$_3$ nach Diamond kultiviert (Diamond, L.S., D.R. Harlow und L.C. Cunnick (1980), A new medium for axenic cultivation of E. histolytica and other Entamoebae, Trans. R. Soc. Trop. Med. Hyg. 72, 431).

i) Nach 72 Stunden werden die Kulturen geerntet, gewaschen und homogenisiert. Anschließend wird das Homogenat niedertourig abzentrifugiert. Der erhaltene Überstand wird nun hochtourig zentrifugiert und der Niederschlag als Membranfraktion verwendet.

j) Mit dem so gewonnenen Antigen wird ein Träger, der vorzugsweise eine Mikrotiterplatte ist, mit einer Pufferlösung beschichtet, z.B. 50 mM $CO_3^{--}/HCO_3^{-}$-Puffer bei 4 °C.

Nachstehend wird ein Beispiel für die Herstellung eines Mittels zur Durchführung des Verfahrens in Form einer Mikrotiterplatte angegeben. Dabei wird wie folgt vorgegangen:

Trophozoiten von Entamoeba histolytica des Stammes A3 werden monoxenisch (Crethidium), wie von Diamond beschrieben, im Medium TYI-S$_3$ (Diamond, L.S., D.R. Harlow und L.C. Cunnick (1980), A new medium for axenic cultivation of E. histolytica and other Entamoebae, Trans. R. Soc. Trop. Med. Hyg. 72, 431) kultiviert. 72 Stunden nach Inokulation werden ca. $2 \times 10^8$ Trophozoiten geerntet und 3 x in 20 ml PBS (Phosphat-Pufferlösung), pH 7,2 (Hersteller: Flow-Laboratories) gewaschen. Dann werden die Trophozoiten in 1 ml PBS suspendiert, mit 100 µl Phenylmethylsulfonylfluorid = PMSF (Hersteller Sigma) (114 mM in i-Propanol) versetzt und mit einem Homogenisator nach Potter-Elvehjem bei 0 °C 2 min homogenisiert. Dann wird bei 4 °C 15 min lang bei 1900 g und der Überstand noch einmal bei 25000 g eine Stunde lang bei 4 °C zentrifugiert. Der Niederschlag ("Membranfraktion") wird in einer Konzentration von 5 µg Protein/ml Karbonat-Puffer I 50 mM $CO_3^{2-}/HCO_3^{-}$ , 0,02 % NaN$_3$, pH 9,6 resuspendiert, und 75 µl davon pro Näpfchen zum Beschichten bei 4 °C für 16 Stunden verwendet.

Danach werden die Näpfchen mit 100 µl PBS-TWEEN (PBS mit 0,05 % TWEEN 20, ein Netzmittel auf Basis von Sorbimacrogollaurat) gewaschen und mit 75 µl PBS-TWEEN, welches 2 % Rinderserumalbumin (BSA, Hersteller: Sigma) enthält, für 16 Stunden bei 4 °C abgesättigt. Danach werden die Näpfchen 3 x mit 100 µl PBS- TWEEN gewaschen und bei -20 °C gelagert.

Weitere Einzelheiten der Erfindung, welche die Früherkennung der extraintestinalen Amöbiasis erlaubt und daher von großem diagnostischem Wert ist, ergeben sich aus der nachstehenden Beschreibung des erfindungsgemäßen amöbenspezifischen ELISA. Mit diesem Verfahren kann die Immunantwort bei invasiver Amöbiasis exakt erfaßt und verfolgt werden.

Es wurden zwei unterschiedliche Antigenfraktionen (Zytoplasma- und Membranfraktion) von Kulturamöben des Stammes A3 hergestellt und die Immunantwort der Patientien gegenüber diesen Fraktionen bestimmt und verglichen. Weiters wurde untersucht, ob eine Aussage über die klassenspezifische Antwort gegenüber Entamoeba histolytica zu treffen und dies von diagnostischem Wert ist.

Material und Methodik

**Amöben:** Trophozoiten von Entamoeba histolytica des Stammes A3 wurden monoxenisch in Medium TYI-S$_3$ nach Diamond kultiviert (Diamond, L.S., D.R. Harlow und C.C. Cunnick (1980), A new medium for the axenic cultivation of E. histolytica and other Entamoebae, Trans. R. Soc. Trop. Med. Hyg. 72, 431). Für alle Antigenpräparationen wurden die Kulturen nach 72 Stunden geerntet.

**Atigenherstellung:** ca. 2 10$^8$ Trophozoiten wurden 3 x in Phosphat-gepuffertem isotonen NaCl, pH 7,2 (PBS) gewaschen, letztlich in 1 - 2 ml PBS resuspendiert und bei -20°C bis zu ihrer Verwendung aufbewahrt. Die aufgetaute Amöbensuspension wurde mit 100 µl PMSF (114 mM Phenylmethylsulfonylfluorid in i-Propanol) versetzt und mit einem Homogenisator nach Potter-Elvehjem bei 0°C 2-3 min homogenisiert. Anschließend wurde das Homogenat 15 min bei 1900 g zentrifugiert (4°C). Der Überstand wurde abgehoben und 1 h bei 25000 g zentrifugiert (4°C). Der Niederschlag wurde als "Membranfraktion", der Überstand als "Zytoplasmafraktion" bezeichnet. Die Proteinbestimmung erfolgte nach Lowry et al. (Lowry, O.H., N.J. Rosenbrough, A.K. Parr & R.J. Randall (1951), Protein measurement with the folin phenol reagent, J. Biol. Chem. 193, 265-275).

Für den ELISA wurden Dynatech (M 129A) Mikrotiterplatten F-Form verwendet. Die Platten wurden mit 75 µl Antigen (5 ug Protein/ml 50 mM CO$_3^{--}$/HCO$_3^-$-Puffer, 0,02 % NaN$_3$, pH 9,6) bzw. nur mit Carbonat Puffer über Nacht bei 4°C beschichtet. Am nächsten Tag wurden die Platten mit PBS 0,05 % TWEEN 20 (PBS-TWEEN) gewaschen und mit 2%-igem BSA in PBS-TWEEN 20 über Nacht bei 4°C abgesättigt. Anschließend wurden die Platten 3 x mit PBS-TWEEN gewaschen. Eine Lagerung bei -20°C war für die Bestimmung von IgA und IgG, nicht aber von IgM Antikörpern möglich.

Die zu untersuchenden Sera wurden mit 2%-iger PBS in PBS-TWEEN 1:200 (IgG, IgM) und 1:40 (IgA) vorverdünnt und in Zweierschritten weiterverdünnt. Hierauf wurden die Platten bei 4°C über Nacht inkubiert. Dann wurde 3 x mit PBS-TWEEN gewaschen.

Fc-spezifisches Anti-human IgG bzw. -IgM von der Ziege, konjugiert mit alkalischer Phosphatase (aP) (Fa. Atlantic Antibodies, Nonesuch Rd, Scarborough, Me. USA) wurde in einer Verdünnung von 1:500 bis 1:800 verwendet. Die Verdünnung erfolgte mit 2%-iger BSA in PBS-TWEEN. Jede neue Charge wurde austitriert und die Arbeitsverdünnung so gewählt, daß der Positivstandard stets den gleichen Titer aufwies. Für den Nachweis von IgA Antikörper wurde ein aP-konjugiertes Anti-human IgA (-kettenspezifisch, Fa. TAGO, INC. Burlingame, CA 94010, USA) in einer Verdünnung von 1:3000 in 2%-iger BSA, in PBS-TWEEN verwendet. 75 µl Konjugatverdünnung wurde zupipettiert; die Platten wurden 2 Stunden bei 37°C belassen. Nach der Inkubation wurden die Platten 3 x mit PBS-TWEEN gewaschen.

Das Substrat wurde kurz vor dem Gebrauch hergestellt. Eine Substrattablette (p-Nitrophenylphosphat 5 mg, Fa. Sigma) wurde in 5 ml 10% Diäthanolaminpuffer, pH 9,8 gelöst. 75 µl dieser Substratlösung wurde zupipettiert und nach 30 min wurde mit 3 M NaOH abgestoppt und die Extinktion bei 405 nm abgelesen (Dynatech).

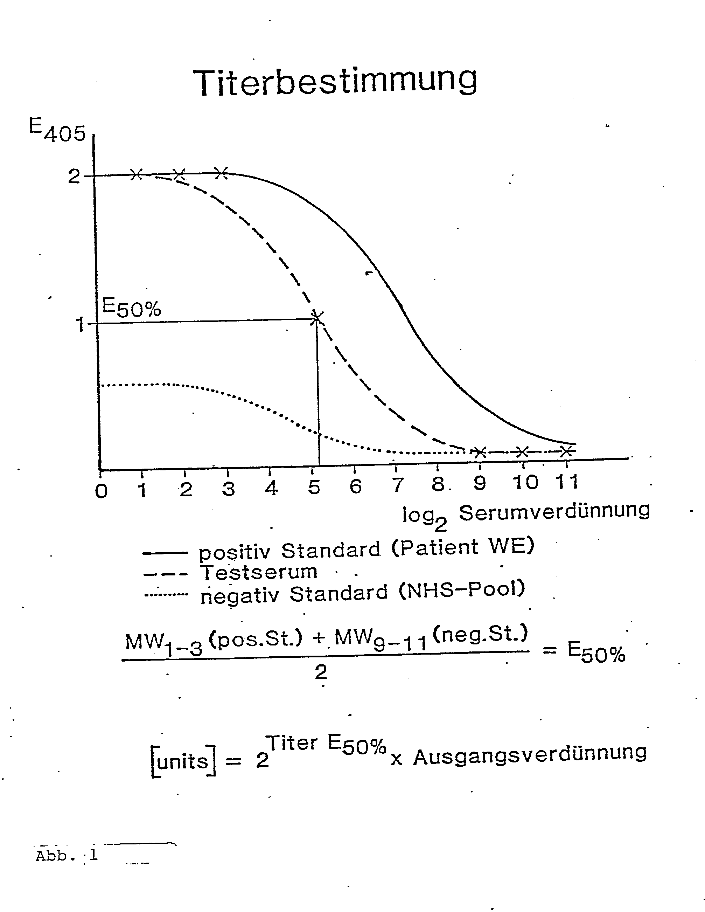

**Titerbestimmung:** (Abb. 1)

Bei jedem Test wurde ein Positivstandard (ein hochtitriges Serum eines Patienten nach akutem Amöben-Leberabszeß) und ein Negativstandard (Normalhumanserum-Pool) mitgeführt. Die Berechnung des Titers wurde folgendermaßen festgelegt:

Es wurde der Mittelwert der Extinktionen der ersten 3 Verdünnungen des Positivstandards sowie der Mittelwert der Extinktionen der letzten Verdünnungen des Negativstandards berechnet und daraus der Mittelwert gezogen (E$_{50\%}$) Nach Aufnahme einer Titrationskurve der Patientensera wurde die Verdünnung des Patientenserums bei E$_{50\%}$ bestimmt. Diese Berechnung hat den Vorteil, daß E$_{50\%}$ von der maximalen Extinktion weitgehend unabhängig ist, und kein willkürlicher Cut-off festgelegt werden muß. Fand sich bei der Kontrolle mit unbeschichteten Näpfen ein signifikanter Titer, so wurde dieser vom Titer bestimmt mit Antigen-beschichteten Näpfen abgezogen. Die Ergebnisse wurden in Units umgerechnet (Abb. 1).

**6 Gruppen von Patienten wurden erfaßt.**

**35 Sera von Patienten mit nachgewiesener invasiver Amöbiasis. (Amöbenpatienten = AP).** Davon waren

13 Sera von 7 Patienten mit akutem Leberabszeß bzw. bis zu zwei Monaten nach Ausbruch der Krankheit,

5 Sera von 4 Patienten, abgenommen 3 - 12 Monate nach der Erstellung der Diagnose Amöben-Leberabszeß,

3 Sera von einem Patienten jeweils 2, 3, 4 Jahre nach Erstellung der Diagnose Amöben-Leberabszeß,

2 Sera eines Patienten mit rezidivem Leberabszeß,

12 Sera von 5 Patienten mit einer intestinalen, invasiven Amöbiasis.

**17 Sera von "nicht-Amöbenpatienten" = NAP.**

14 Sera von 12 Patienten mit parasitären Infektionen (Echinococcosis, Schistosomiasis, Toxocariose, Toxoplasmose, Fascioliasis, Zystizerkose), 2 Rheumafaktor positiv, 1 SLE = NAP

**21 Sera von Kindern ohne parasitäre Infektion = Ki**

Diese Sera stammten vom Preyerschen Kinderspital.

6 Kinder ( 4 Monate - 1 Jahr)

6 Kinder (1 Jahr - 3 Jahre)

8 Kinder (3 - 14 Jahre)

**12 Sera von schwangeren Frauen ohne manifeste Infektion = GR**

(Test auf Toxoplasmose)

**19 Personen, die auf Tropentauglichkeit untersucht wurden und die nach eigenen Angaben Europa nie verlassen hatten = TT**

**129 Tropenrückkehrer = TR** (die sich einer routinemäßigen Tropenrückkehreruntersuchung unterzogen hatten).

Ergebnisse und Diskussion

IgG-Antwort gegen E. histolytica

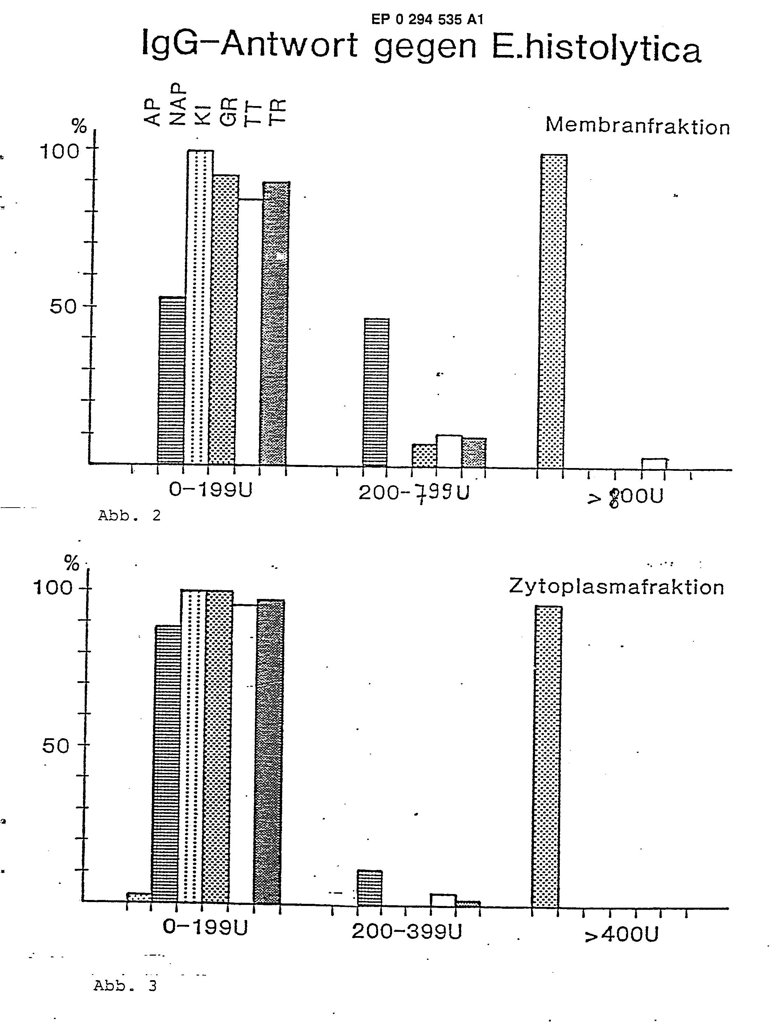

Die Sera von Personen der Gruppe AP (Amöbenpatienten, s.o.) wiesen sowohl gegenüber der Membranfraktion (im Mittel 6100 U) wie gegenüber der Zytoplasmafraktion (im Mittel 2100 U) hohe Titer in der IgG Klasse auf (Abb. 2 u. 3). Bis zu 4 Jahren nach Erstellung der Diagnose konnte bei einem Patienten ein signifikanter Titer von 1600 U in der Membranfraktion und von 800 U in der Zytoplasmafraktion beobachtet werden. Bei Verwendung der Membranfraktion wiesen alle Patienten der Gruppe AP einen Titer ≥ 1600 U auf; bei Verwendung der Zytoplasmafraktion zeigte ein Serum unter 200 U (Membranfraktion 2000 U).

Dieses Serum stammte von einer Patientin, bei der einige Tage später ein Amöben-Leberabszeß diagnostiziert wurde. Serumproben dieser Patientin zeigten, daß schon nach einigen Tagen IgG Antikörper (800 U) gegenüber der Zytoplasmafraktion gebildet wurden.

Ein einziges Serum, das nicht von einem Patienten mit nachgewiesener invasiver Amöbiasis stammte, wies einen signifikanten Titer (880 U) in der Membranfraktion auf. Dieses Serum stammte von einem Patienten, der sich einer Tropentauglichkeitsuntersuchung unterzogen hatte. Er wies einen erhöhten Titer in IgG und IgM auf (Tab. 1). Der Proband hatte zur Zeit der Serumabnahme nach eigenen Angaben keinerlei Beschwerden. 1974 hatte der Patient an Gastritis gelitten. Eine jetzt durchgeführte Blutchemie ergab lediglich ein erhöhtes Gesamt-IgA (4,7 mg/ml). Serologische Untersuchungen, die am parasitologischen Institut Wien durchgeführt wurden, ergaben Amöben IHA-negativ, Amöben IFT 1:32.

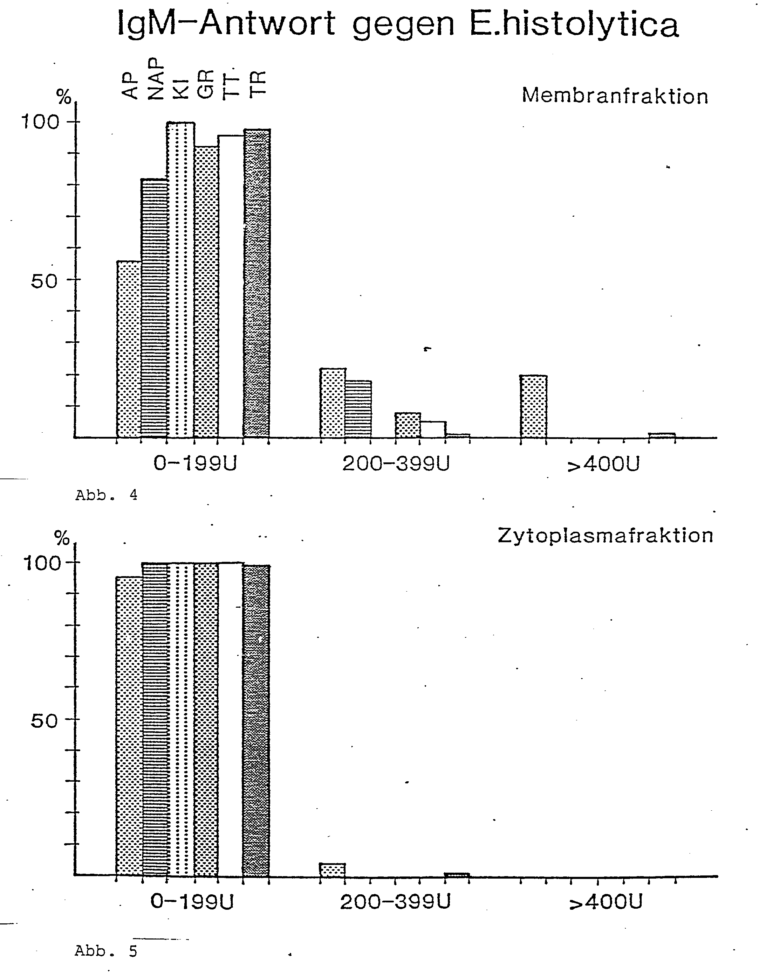

47 % der Gruppe Patienten mit einer parasitären Infektion (NAP) erreichten im IgG einen Titer zwischen 200 und 500 U mittels der Membranfraktion. Es könnte sich hierbei um Kreuzreaktionen mit anderen Parasitenantigenen handeln, man kann diese Titer aber auch im Sinne eines polyklonalen Stimulus infolge der parasitären Infektion erklären. Diese offenbar nicht amöbenspezifischen Reaktionen sind gegenüber der Zytoplasmafraktion kaum zu beobachten (Abb. 3).

Mit Hilfe der oben angeführten Daten wurde bei Verwenden von Membranfraktionen eine Signifikanzgrenze von 800 ≥ U, bei Verwendung von Zytoplasmafraktion eine solche von ≥ 400 U festgelegt.

IgM-Antwort gegen E. histolytica

Die Immunantwort in der Klasse IgM ist deutlich schwächer (Tabelle); signifikante Titer ( ≥ 400 U), lassen sich nur bei Verwendung von Membranantigen beobachten (Abb. 4 und 5). 20 % der Sera der Gruppe AP wiesen einen IgM Titer von mehr als 400 U auf, 22,9 % hatten einen Titer zwischen 200 und 399 U und 57,1 % hatten keinen signifikanten Titer (unter 200 U). In die letzte Kategorie fallen Sera eines Patienten mit rezidivem Leberabszeß, solche von Patienten zwei Monate bis vier Jahre nach Erstellung der Diagnose Amöbenleberabszeß, solche von Patienten, die einmal eine Amöbencolitis durchgemacht hatten und zuletzt ein Serum eines Patienten mit akutem Leberabszeß (Abb. 6). Dieser Patient zeigte 3 Tage später IgM-Antikörper (230 U) gegen Entamoeba histolytica.

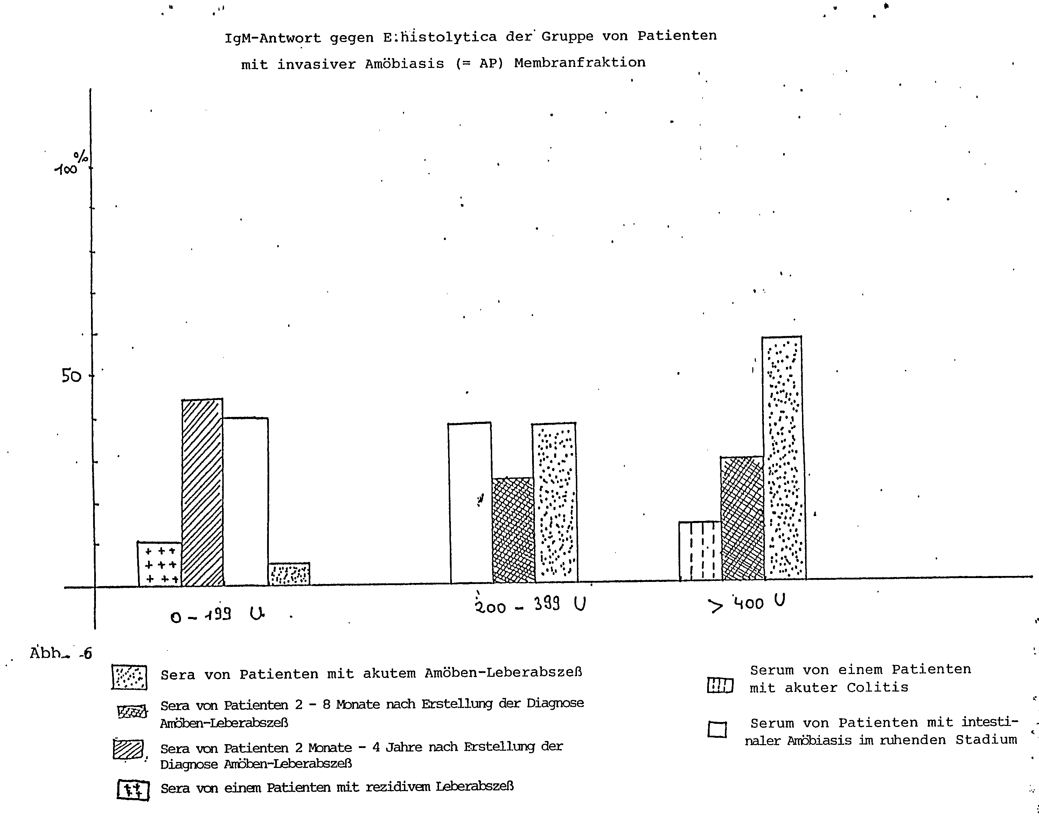

Signifikante Titer ( ≧ 400 U) wiesen 57 % der Sera von Patienten mit akutem Amöbenleberabszeß, 29 % der Sera von Patienten 2 - 8 Monate nach der Erstellung der Diagnose Amöben-Leberabszeß und ein Serum (14 %) eines Patienten mit akuter Amöbencolitis (Abb. 6). Es muß darauf hingewiesen werden, daß die Sera von Patienten mit akutem Amöben-Leberabszeß einen Titer von 1000 U erreichten, wogegen das Serum eines Patienten mit akuter Amöbencolitis nur 460 U aufwies.

Es ist empfehlenswert, für jedes Serum eine Kontrolle mit Antigen-unbeschichteten Näpfen mitzuführen. Insbesondere wichtig ist dies bei Nachweis von anti-Amöben IgM.

Es sei erwähnt, daß offenbar niedere Titer gegen Entamoeba histolytica (20 U - 60 U, IgM und IgG) bei Normalpersonen (gesundes Laborpersonal ohne jede einschlägige Anamnese) immer zu beobachten sind. Diese "natürlichen" Antikörper erkennen auch andere Antigene als jene von Amöbenpatienten mit hohen Titern, wie mittels Westernblot-Technik nachgewiesen werden konnte.

IgA-Antwort gegen Entamoeba histolytica

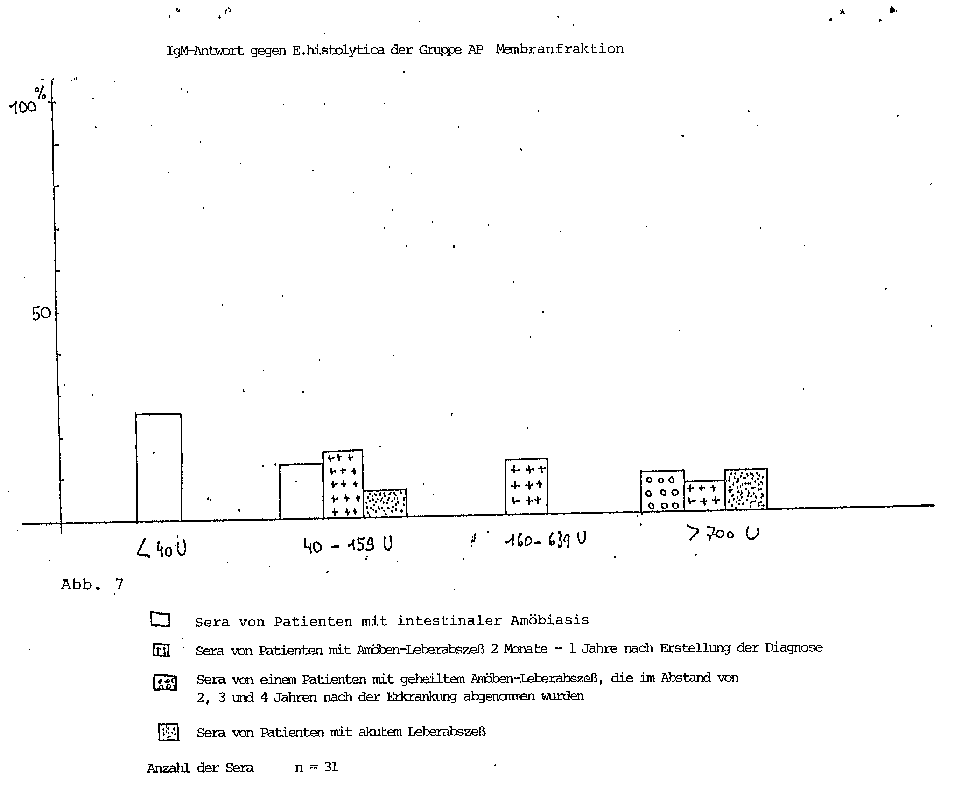

Die verhältnismäßig schwache Immunantwort der Gruppe AP in der Klasse IgA (mittlerer Titer 400 U) ähnelt jener in der Klasse IgM: signifikante Titer sind nur gegenüber der Membranfraktion feststellbar (Tabelle). Auffallend ist die Tatsache, daß lediglich Patienten mit einem akuten bzw. abgeheilten Amöbenleberabszeß signifikante Titer erreicht werden (Abb. 7).

Nur 2 Sera von 2 Patienten mit akutem Amöbenleberabszeß hatten einen Titer von 40 - 160 U (Abb. 7). Einer der beiden Patienten entwickelte innerhalb von 3 Tagen IgA-Antikörper mit einem Titer von 3800 U (während die IgM-Antwort eher schwach blieb: 230 U). Der andere Patient hatte wohl eine niedrige Immunantwort in der Klasse IgA, die IgM-Antwort war jedoch sehr stark (mehr als 1000 U).

Vergleich der serologischen Methoden

IHA und IIFT wurden dann als positiv bewertet, wenn sie einen Titer von 1:128 aufwiesen. Es zeigte sich, daß Sera von Patienten mit anderen parasitären Infektionen (z.B. Shistosoma haematobium, Shistosoma mansoni und Echinococcus) im Indirekthaemagglutinationstest (IHA) falsch positiv bewertet wurden. Die Titer reichten von 1:2000 bis 1:32000. Ein Serum eines Patienten mit Echinococcus wurde im IFT 1:512 falsch positiv bewertet, ferner ein SLE Serum mit 1:160. Die ELISA-Ergebnisse waren sehr zufriedenstellend.

Bei 7 Patienten mit akutem Amöben-Leberabszeß wiesen 57 % der Sera einen signifikanten IgM-Titer (> 400 U) auf und 28,6 % einen signifikanten IgA-Titer (> 1000 U) und nur ein Serum hatte unverdächtige Ergebnisse in der IgM- bzw. IgA-Bestimmung. Auffallend war, daß, wenn eine hohe Immunantwort in der IgM-Klasse erfolgt war, die Immunantwort in der IgA-Klasse schwächer ausfiel (< 1000 U), auf der anderen Seite jedoch, wenn eine hohe Immunantwort in der IgA- Klasse erfolgte, die IgM-Antwort keine Signifikanzgrenze erreichte. Es ist immer wieder darauf hinzu weisen, daß eine verläßliche IgM- und IgA-Bestimmung besonders mit der Membranfraktion als Antigen möglich ist. Somit empfiehlt es sich, neben einer IgG-Bestimmung auch zusätzlich eine IgM- bzw. IgA-Bestimmung als Kontrolle mitzuführen, was keine sonderliche Mehrarbeit im Labor erfordert. Die anderen serologischen Methoden (KBR, IHA, Latex) sind, jede für sich allein nicht genügend aussagekräftig. So benötigen viele Labors unterschiedliche Untersuchungen, um Amöbenantikörper deuten zu können; diese Arbeitsweise ist sicherlich weit aufwendiger und kostspieliger.

Es zeigt sich, daß eine hohe Immunantwort bei Patienten mit invasiver Amöbiasis in der Klasse IgG erreicht wird, und zwar sowohl gegenüber der Membranfraktion wie gegenüber der Zytoplasmafraktion. Die Titer sind in der Membranfraktion wesentlich höher als in der Zytoplasmafraktion.

Die Immunantwort in der Klasse IgM und IgA ist schwächer; signifikante Titer lassen sich nur bei Verwendung von Membranantigen beobachten. Für die IgM-Bestimmung ist eine Kontrollführung für jedes Serum empfehlenswert. Signifikante IgA-Titer wurden nur bei Patienten mit Amöbenleberabszeß gefunden.

## TABELLE

## ANTIKÖRPER GEGEN E. HISTOLYTICA

| | MEMBRAN-FR. | | ZYTOPLASMA-FR. | |
|---|---|---|---|---|
| | IgG | | | |
| | UNITS (MW) | RANGE | UNIT (MW) | RANGE |
| AP | 6.100 | 1.100-40.000 | 2.100 | 0-40.000 |
| NAP | 180 | 0-420 | <200 | 0-280 |
| KI | 0 | | 0 | |
| GR | <200 | 0-320 | 0 | |
| TT | <200 | 0-880 | <200 | 0-260 |
| TR | <200 | 0-760 | <200 | 0-420 |
| | IgM | | | |
| AP | < 40 | 0-1.400 | <200 | 0-400 |
| NAP | <200 | 0-320 | 0 | |
| KI | 0 | | 0 | |
| GR | 0 | | 0 | |
| TT | <200 | 0-200 | 0 | |
| TR | <200 | 0-720 | <200 | 0-200 |
| | IgA | | | |
| AP | 400 | 0-3.800 | <200 | 0-800 |
| NAP | < 40 | 0-50 | 0 | |
| KI | | N.D. | | N.D. |
| GR | 0 | | 0 | |
| TT | 0 | | 0 | |
| TR | | N.D. | | N.D. |

## Ansprüche

1. Verfahren zum Nachweis von Antikörpern gegen Entamoeba histolytica in Proben, insbesondere in Blutproben, gekennzeichnet durch folgende Verfahrensschritte:

a) Kontaktieren der Probe mit Entamoeba histolytica-Antigen, das auf einem Träger fixiert ist,

b) Entfernen derjenigen Antikörper, die mit dem fixierten Entamoeba histolytica-Antigen keine Antikörper-Antigen-Reaktion eingegangen sind,

c) Kontaktieren des so behandelten Trägers mit einem Antikörper, insbesondere einem Anti-Human Immunglobulin, der mit einem Enzym z.B. mit alkalischer Phosphatase konjugiert ist und der sich ausschließlich an den Antigen-Antikörper-Komplex anlagert,

d) Entfernen des nicht angelagerten, mit dem Enzym konjugierten Antikörpers,

e) Reaktion des Enzyms mit einem Substrat, um eine Farbreaktion auszulösen,

f) Abstoppen der Reaktion und

g) Prüfen der so behandelten Probe hinsichtlich des Auftretens einer Verfärbung.

2. Verfahren nach Anspruch 1, dadurch gekennzeichnet, daß man in Stufe c) mit Meerrettich-Peroxidase oder Glukose-6- Phosphatase konjugiertes Anti-Human Immunglobulin verwendet.

3. Verfahren nach Anspruch 1 oder 2, dadurch gekenn zeichnet, daß man in Stufe c) ein Anti-Human IgG, IgM oder IgA verwendet.

4. Verfahren nach einem der Ansprüche 1 bis 3, dadurch gekennzeichnet, daß man als Substrat in Stufe e) o-Phenylendiamin. 2HCl oder p-Nitrophenylphosphat verwendet.

5. Mittel zur Durchführung des Verfahrens nach einem der Ansprüche 1 bis 4, dadurch gekennzeichnet, daß es aus einem Träger, der vorzugsweise eine Mikrotiterplatte ist, besteht, der mit einem Entamoeba histolytica-Antigen beschichtet ist.

6. Verfahren zur Herstellung eines Mittels nach Anspruch 5, dadurch gekennzeichnet, daß man Trophozoiten von Entamoeba histolytica, insbesondere des Stammes A3 kultiviert, daß man aus der Kulturbrühe Entamoeba histolytica-Antigen gewinnt und mit dem gewonnenen Antigen einen Träger beschichtet.

7. Verfahren nach Anspruch 6, dadurch gekennzeichnet, daß man die Trophozoiten von Entamoeba histolytica monoxenisch, insbesondere mit Crethidium in einem Medium, vorzugsweise im Medium TYI-S$_3$ nach Diamond kultiviert.

8. Verfahren nach Anspruch 6 oder 7, dadurch gekennzeichnet, daß man die Kulturbrühe homogenisiert und zentrifugiert unbd daß man den Träger mit dem derart gewonnenen Niederschlag (Membranfraktion), nachdem dieser in einer Pufferlösung, vorzugsweise einen 50 mM $CO_3^{--}$/$HCO_3^-$ - Puffer, aufgenommen worden ist, beschichtet.

# Titerbestimmung

$$\frac{MW_{1-3} \, (\text{pos.St.}) + MW_{9-11} \, (\text{neg.St.})}{2} = E_{50\%}$$

$$[\text{units}] = 2^{\text{Titer } E_{50\%}} \times \text{Ausgangsverdünnung}$$

Abb. 1

# IgG-Antwort gegen E.histolytica

Membranfraktion

AP NAP KI GR TT TR

%
100
50

0-199U        200-799U        > 800U

Abb. 2

Zytoplasmafraktion

%
100
50

0-199U        200-399U        >400U

Abb. 3

# IgM-Antwort gegen E.histolytica

Membranfraktion

Abb. 4

Zytoplasmafraktion

Abb. 5

IgM-Antwort gegen E.histolytica der Gruppe von Patienten

mit invasiver Amöbiasis (= AP) Membranfraktion

Abb. 6

| | Sera von Patienten mit akutem Amöben-Leberabszeß |
| | Sera von Patienten 2 - 8 Monate nach Erstellung der Diagnose Amöben-Leberabszeß |
| | Sera von Patienten 2 Monate - 4 Jahre nach Erstellung der Diagnose Amöben-Leberabszeß |
| | Sera von einem Patienten mit rezidivem Leberabszeß |

Serum von einem Patienten mit akuter Colitis

Serum von Patienten mit intestinaler Amöbiasis im ruhenden Stadium

EP 0 294 535 A1

IgM-Antwort gegen E.histolytica der Gruppe AP Membranfraktion

Abb. 7

☐  Sera von Patienten mit intestinaler Amöbiasis

▦  Sera von Patienten mit Amöben-Leberabszeß 2 Monate – 1 Jahre nach Erstellung der Diagnose

▦  Sera von einem Patienten mit geheiltem Amöben-Leberabszeß, die im Abstand von
   2, 3 und 4 Jahren nach der Erkrankung abgenommen wurden

▦  Sera von Patienten mit akutem Leberabszeß

Anzahl der Sera      n = 31

EP 0 294 535 A1

Europäisches
Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | ANN. SOC. BELGE MED. TROP., Band 62, 1982, Seiten 103-120; F. SPEISER: "Serodiagnosis of tissue dwelling parasites: application of a multi-antigen enzyme-linked immunosorbent assay (ELISA) for screening" * Seite 103, Zeile 1 - Seite 107, Zeile 30 * | 1-6 | G 01 N 33/569 C 12 N 11/00 C 12 N 1/10 |
| A | Idem --- | 7,8 | |
| X | ANN. NATL. ACAD. MED. SCI. (INDIA), Band 22, Nr. 1, Januar-März 1986, Seiten 18-25; M. IRSHAD et al.: "An improved ELISA technique to detect amoebic antibody in blood" * Seiten 18-20 * --- | 1-4,6,7 | |
| X | JOURNAL OF CLINICAL MICROBIOLOGY, Band 13, Nr. 4, April 1981, Seiten 646-651; T.M. LIN et al.: "Simple standardized enzyme-linked immunosorbent assay for human antibodies to Entamoeba histolytica" * Insgesamt * | 1,3,4,6 ,8 | RECHERCHIERTE SACHGEBIETE (Int. Cl.4) G 01 N C 12 N |
| A | Idem --- | 7 | |
| X | JOURNAL OF IMMUNOLOGICAL METHODS, Band 83, 1985, Seiten 125-133, Elsevier Science Publishers B.V., New York, US; KUMAR et al.: "A dot enzyme-linked immunosorbent assay for detection of antibodies against Entamoeba histolytica" * Insgesamt *               -/- | 1,2,5,6 ,8 | |

Der vorliegende Recherchenbericht wurde für alle Patentansprüche erstellt

| Recherchenort | Abschlußdatum der Recherche | Prüfer |
|---|---|---|
| DEN HAAG | 10-02-1988 | HITCHEN C.E. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus andern Gründen angeführtes Dokument
.........................................................
& : Mitglied der gleichen Patentfamilie, übereinstimmendes
   Dokument

EPO FORM 1503 03.82 (P0403)

Europäisches Patentamt

EUROPÄISCHER RECHERCHENBERICHT

Nummer der Anmeldung

EP 87 89 0127

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | Idem<br>--- | 7 | |
| X | DE-A-3 346 795 (E. GEYER)<br>* Insgesamt *<br>--- | 1-5 | |
| A | INDIAN J. MED. RES., Band 80, November 1984, Seiten 528-531; U.K. BAVEJA et al.: "Enzyme-linked immunosorbent assay in amoebiasis"<br>* Insgesamt *<br>--- | 1-8 | |
| A | US-A-3 951 748 (R.F. DEVLIN)<br>* Figuren 1,2; Spalte 6, Zeilen 21-29; Spalte 8, Zeilen 35-57; Spalte 9, Zeile 55 - Spalte 11, Zeile 42; Ansprüche 18,21,22 *<br>--- | 1-5 | |
| A | CHEMICAL ABSTRACTS, Band 89, Nr. 23, 4. Dezember 1978, Seite 312, Zusammenfassung Nr. 193750p, Columbus, Ohio, US; L.S. DIAMOND et al.: "A new medium for the axenic cultivation of Entamoeba histolytica and other Entamoeba", & TRANS. R. SOC. TROP. MED. HYG. 1978, 72(4), 431-2<br>* Zusammenfassung *<br>--- | 7 | RECHERCHIERTE SACHGEBIETE (Int. Cl.4) |
| A | BIOLOGICAL ABSTRACTS, Band 83, Nr. 7, 1987, Seiten 809,810, Zusammenfassung Nr. 67865, Philadelphia, PA, US; I. MEZA et al.: "Isoenzyme patterns of Entamoeba histolytica isolates from asymptomatic carriers: Use of gradient acrylamide gels", & AM. J. TROP. MED. HYG. 1986, 35(6), 1134-1139<br>* Zusammenfassung *<br>----- | 7 | |

Der vorliegende Recherchenbericht wurde für alle Patentansprüche erstellt

| Recherchenort | Abschlußdatum der Recherche | Prüfer |
|---|---|---|
| DEN HAAG | 10-02-1988 | HITCHEN C.E. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
............................................................
& : Mitglied der gleichen Patentfamilie, übereinstimmendes Dokument

EPO FORM 1503 03.82 (P0403)